# EUROPÄISCHE PATENTSCHRIFT

(11) **EP 3 464 552 B1**
(45) Veröffentlichungstag und Bekanntmachung des Hinweises auf die Patenterteilung: **13.04.2022**
(21) Anmeldenummer: 17726917.2
(22) Anmeldetag: 26.05.2017
(51) Int. Cl.: C12M 3/00, C12M 1/22, C12M 1/12, C12M 1/34, C12M 1/42

(54) **LOCHPLATTE**
PERFORATED PLATE
PLAQUE PERFORÉE

(30) Priorität: 03.06.2016 DE 102016110328
(43) Veröffentlichungstag der Anmeldung: 10.04.2019
(73) Patentinhaber: Georg-August-Universität Göttingen Stiftung Öffentlichen Rechts, Universitätsmedizin, 37075 Göttingen (DE)
(72) Erfinder: MEYER, Tim, 37083 Göttingen (DE); ZIMMERMANN, Wolfram-Hubertus, 37073 Göttingen (DE); TIBURCY, Malte, 37083 Göttingen (DE)
(74) Vertreter: REHBERG HÜPPE + PARTNER
(86) Internationale Anmeldenummer: PCT/EP2017/062755
(87) Internationale Veröffentlichungsnummer: WO 2017/207431

(56) Entgegenhaltungen:
- WO-A1-2007/054286
- DE-A1- 10 003 521
- US-A1- 2009 068 701

## Beschreibung

### TECHNISCHES GEBIET DER ERFINDUNG

Die Erfindung bezieht sich auf eine Lochplatte mit einem Plattenhauptkörper und mit mindestens einer Vertiefung in einer Oberseite des Plattenhauptkörpers, wobei in der Vertiefung ein oben offener Ringkanal ausgebildet ist, der an seinem Innenumfang durch eine umlaufende Wandung begrenzt ist.

Insbesondere bezieht sich die Erfindung auf eine Lochplatte mit mehreren solchen Vertiefungen zur Herstellung und Untersuchung von Ringen aus künstlichem durch sogenanntes Tissue Engineering hergestelltes Gewebe; z.B. Herzmuskel- und Skelettmuskelgewebe und Bindegewebe, wobei der Begriff Ring so zu verstehen ist, dass das jeweilige Gewebe eine geschlossene Schlaufe oder Schleife ausbildet, nicht aber so, dass diese Schlaufe oder Schleife eine besondere geometrische Form haben oder gar kreisrund sein muss.

Weiterhin bezieht sich die Erfindung auf eine Verwendung einer solchen Lochplatte insbesondere im Rahmen der Herstellung und Untersuchung von Ringen aus künstlichem Herzmuskel- und Skelettmuskelgewebe.

Grundsätzlich können die Lochplatte und ihre Verwendung aber auch zur Herstellung und Untersuchung von Ringen aus anderen Zellen bzw. Zellgemischen, wie beispielsweise aus Zellen des Bindegewebes oder Organzellen wie z.B. Neuronen, Hepatozyten oder aus anderen Substanzen wie beispielsweise elektro- oder magnetorheologischen Polymeren, verwendet werden.

Der Begriff "Lochplatte" ist in der gesamten vorliegenden Beschreibung und den anhängenden Patentansprüchen nicht als Einschränkung zu verstehen. So deckt er grundsätzlich beispielsweise auch Kulturschalen mit mindestens einer Vertiefung ab, soweit die mindestens eine Vertiefung die Merkmale aufweist, wie sie hier für die mindestens eine Vertiefung der Lochschale definiert sind.

### STAND DER TECHNIK

Eine Kulturschale mit einer Vertiefung, in der durch Einsetzen eines zentralen Zylinders aus hydrophobem Material ein Ringkanal ausgebildet ist, der an seinem Innenumfang durch den Außenumfang des Zylinders begrenzt ist, ist aus der DE 100 03 521 A1 bekannt. Wenn eine spezielle Suspension eines Hydrogels und von Herzmuskelzellen in den Ringkanal gegeben wird, bildet sich ein künstliches Herzmuskelgewebe in Form eines den Zylinder umschließenden Rings.

Gemäß der WO 2007/054286 A1 kann dieser Ring von dem Zylinder abgenommen und auf zwei parallel zueinander angeordneten Haltefingern, sogenannten Stretchern, angeordnet werden, die in Richtung ihres Abstands elastisch aneinander abgestützt sind. Auf diesen Stretchern reift der Ring aus künstlichem Herzmuskelgewebe in Nährlösung und beginnt selbsttätig mit periodischen Kontraktionen mit einer typischen Herzfrequenz.

In der bekannten Kulturschale mit dem zentralen, vertikal ausgerichteten Zylinder kann auch künstliches Skelettmuskelgewebe durch Einfüllen einer speziellen Suspension von Hydrogel und Skelettmuskelzellen generiert werden. Der dabei entstehende Ring aus künstlichem Skelettmuskelgewebe ist zur Reifung ebenfalls in Nährlösung auf Stretchern anzuordnen. Er zeigt aber keine rhythmischen Kontraktionen, sondern ist hierfür elektrisch zu reizen.

In der bekannten Kulturschale mit dem zentralen, vertikal ausgerichteten Zylinder kann auch künstliches Bindegewebe durch Einfüllen einer speziellen Suspension von Hydrogel und Bindegewebszellen hergestellt werden. Der dabei entstehende Ring aus künstlichem Bindegewebe ist zur Reifung ebenfalls in Nährlösung auf Stretchern anzuordnen. Er ist nicht zu rhythmischen Kontraktionen befähig, sondern zieht die Stretcher tonisch in Abhängigkeit der Gewebespannung zusammen.

Das Umsetzen der Ringe aus künstlichem Gewebe von dem zentralen Zylinder in der Vertiefungen der Kulturschale ist aufwändig und nicht ohne weiteres automatisierbar. Umfangreiche Untersuchungen an Ringen aus künstlichem Gewebe sind so nicht sinnvoll möglich.

Aus der WO 2014/154704 A1 ist eine Vorrichtung zum Messen der Kontraktionseigenschaften von künstlichem Herzmuskelgewebe bekannt. Dazu wird eine Suspension eines Hydrogels und von Herzmuskelzellen in Wasser in eine Kulturschale eingebracht. In die Suspension werden voneinander weg nach außen abgebogene untere Enden von zwei im Wesentlichen vertikal ausgerichteten Haltefingern eingetaucht. Hieran lagert sich unter geeigneten Bedingungen das polymerisierende Hydrogel mit darin eingebetteten Herzmuskelzellen aus der Suspension derart an, dass es ein knochenförmiges Konstrukt ausbildet, das die freien Enden der Haltefinger miteinander verbindet. Mit der Vorrichtung wird das Konstrukt aus der Vertiefung der Lochplatte entfernt und in eine Nährlösung in einer Vertiefung einer anderen Lochplatte überführt. Kontraktionsbewegungen dieses Konstrukts werden durch piezoelektrische Wandler erfasst, über die die Haltefinger an einer Basis der Vorrichtung gelagert sind. Die Vorrichtung und Ihre Verwendung sind aufwändig und störanfällig und daher für umfangreiche Untersuchungen einer Vielzahl von Gewebekonstrukten unter verschiedenen Randbedingungen nicht sinnvoll einsetzbar.

Eine der aus der WO 2014/154704 A1 bekannten Vorrichtung ähnliche Vorrichtung ohne piezoelektrische Wandler ist bereits aus A. Hansen et al.: Development of a screening platform based on engineered heart tissue, Circ Res 107: 35-44, 2010 bekannt. Hier sind die Haltefinger elastisch an der Basis der Vorrichtung abgestützt und Kontraktionsbewegungen des die Haltefinger verbindenden knochenförmigen Konstrukts werden durch optische Beobachtung der resultierenden Bewegung der Haltefinger mit Hilfe einer Kamera erfasst. Konkret wird die Änderung des Abstands der einander gegenüberliegenden vertikalen Oberflächen der Haltefinger beobachtet. Zuvor werden in mehreren Löchern einer Lochplatte, deren horizontaler Querschnitt auf eine gewünschte Form des Konstrukts abgestimmt ist, gleichzeitig mehrere Konstrukte jeweils zwischen den unteren Enden eines Paars von Haltefingern ausgebildet und dann mit der Vorrichtung in mit Nährlösung befüllte Löcher einer weiteren Lochplatte überführt.

Aus der WO 2015/061907 A1 ist eine Lochplatte mit einer Vielzahl von Löchern zum Herstellen jeweils eines Strangs aus künstlichem Muskelgewebe bekannt. In jeder Vertiefung der Lochplatte sind an einem Plattenhauptkörper der Lochplatte befestigte Gerüstelemente angeordnet, die dazu vorgesehen sind, in den jeweiligen Strang eingebettet zu werden. Bei Kontraktion des Strangs bewegen sich die darin eingebetteten Gerüstelemente. Die Kontraktion des Strangs kann mit Hilfe eines Paars von Elektroden angeregt werden, die an den Gerüstelementen angeordnet sind. In der bekannten Lochplatte sollen die Auswirkungen von verschiedenen Substanzen wie Therapeutika und Toxinen auf die Kontraktion des Strangs untersucht werden. Bei der bekannten Lochplatte beeinflussen die eingebetteten Gerüststrukturen die Eigenschaften des Gewebestrangs.

### AUFGABE DER ERFINDUNG

Der Erfindung liegt die Aufgabe zugrunde, eine Lochplatte und ein Verfahren zu deren Verwendung aufzuzeigen, mit denen ohne großen Aufwand umfangreiche Untersuchungen an einer großen Zahl von Ringen aus künstlichem Herzmuskel- und Skelettmuskel- sowie anderen Gewebearten parallel und automatisiert durchgeführt werden können.

### LÖSUNG

Die Aufgabe der Erfindung wird durch eine Lochplatte mit den Merkmalen des Patentanspruchs 1 und ein Verfahren zur Verwendung der Lochplatte mit den Merkmalen des Patentanspruchs 16 gelöst. Bevorzugte Ausführungsformen der Lochplatte und der erfindungsgemäßen Verwendung sind in den abhängigen Patentansprüchen definiert.

### BESCHREIBUNG DER ERFINDUNG

Bei einer erfindungsgemäßen Lochplatte mit einem Plattenhauptkörper und mit mindestens einer Vertiefung in einer Oberseite des Plattenhauptkörpers, wobei in der mindestens einen Vertiefung ein oben offener Ringkanal ausgebildet ist, der an seinem Innenumfang durch eine geschlossen umlaufende Wand begrenzt ist, nimmt ein horizontaler Außenumfang der umlaufenden Wand von unten nach oben bis an eine Oberkante der umlaufenden Wand ab und innerhalb des horizontalen Außenumfangs der umlaufenden Wand an ihrer Oberkante schließen mindestens zwei Halteelemente nach oben an die Oberkante der umlaufenden Wand an. Dabei weisen die mindestens zwei Halteelemente einen freien horizontalen Abstand voneinander auf, und mindestens eines der mindestens zwei Halteelemente ist in horizontaler Richtung elastisch an dem Plattenhauptkörper abgestützt.

Die Bezeichnung Ringkanal ist hier so zu verstehen, dass sie keine besondere Form, insbesondere keine Kreisform des so bezeichneten Kanals oder der ihn an seinem Innenumfang begrenzenden geschlossen umlaufenden Wand fordert. Vielmehr deckt diese Bezeichnung jeden kontinuierlichen sowie in sich geschlossenen Kanal ab. Der Querschnitt des Ringkanals kann dabei über seinen Innenumfang konstant sein. Dies ist aber nicht zwingend.

Wenn in den Ringkanal in der mindestens einen Vertiefung der erfindungsgemäßen Lochplatte eine Suspension, von Bindegewebszellen, wie beispielsweise Fibroblasten, und von weiteren Zellen, wie beispielsweise Herzmuskelzellen, eingegeben wird, die zudem ein Hydrogel aufweisen kann und aus der sich ein an die umlaufende Wandung anlegender Ring bildet, wandert dieser Ring durch den nach oben abnehmenden Außenumfang des Rings selbsttätig nach oben. Die Geschwindigkeit dieses Wanderns des Rings nach oben hängt von der Spannung des Rings ab, mit der er sich an die umlaufende Wand anlegt, von der Ableitung des horizontalen Außenumfangs der Wand, nach der Höhe über dem Grund des Ringkanals und von der Haftreibung zwischen dem Ring und der umlaufenden Wand. Das Wandern des Rings nach oben setzt sich so lange fort, bis der Ring die Oberkante der Wand überschreitet und auf die oben an die Wand anschließenden Halteelemente übertritt. Diese, mindestens zwei Halteelemente nehmen den Ring auf. Dadurch dass mindestens eines von ihnen in horizontaler Richtung elastisch an dem Plattenkörper abgestützt ist, sind sie aneinander elastisch abgestützt und erfüllen die Funktion von Stretchern für den Ring. Bei Verwendung der erfindungsgemäßen Lochplatte entfällt so die Notwendigkeit, einen in dem Ringkanal gebildeten, an der den Innenumfang des Ringkanals bildenden umlaufenden Wand anliegenden Ring von der Wand abnehmen und auf Stretcher überführen zu müssen. Diese Überführung erfolgt vielmehr selbsttätig mit dem Zusammenziehen des Rings durch den nach oben abnehmenden horizontalen Außenumfang der umlaufenden Wand. Dazu muss der horizontale Außenumfang der umlaufenden Wand weder stetig noch streng monoton nach oben abnehmen, auch wenn dies bevorzugt ist.

Bei der neuen Lochplatte können die Halteelemente grundsätzlich an einem gegenüber dem Grundkörper der Lochplatte beweglichen weiteren Körper abgestützt sein. Vorzugsweise ist jedoch jedes der mindestens zwei Halteelemente in der mindestens einen Vertiefung an dem Plattenhauptkörper abgestützt. Dadurch weist die Lochplatte einen insgesamt einfachen Aufbau auf. Es tritt auch eine Vereinfachung bei ihrer Verwendung auf, weil ein in einer Vertiefung der Lochplatte hergestellter Ring für seine Untersuchung nicht aus der Vertiefung entnommen werden muss. Vielmehr ist die Untersuchung des jeweiligen Rings in derselben Vertiefung auf den Halteelementen oberhalb der Oberkante der umlaufenden Wand vorgesehen.

Bei dieser Ausführungsform der erfindungsgemäßen Lochplatte ist also zumindest das elastisch an dem Plattenkörper abgestützte Halteelement Teil eines solchen Haltefingers. Aufgrund des Haltefingers ist das jeweilige Halteelement in einfacher Weise weich elastisch abgestützt. Dies gilt sowohl dann, wenn der jeweilige Haltefinger seinerseits elastisch verformbar ist, als auch dann, wenn der Haltefinger zwar formsteif aber elastisch gelagert ist. In beiden Fällen steigt die Weichheit der Abstützung des jeweiligen Halteelements mit der Länge des Haltefingers zwischen dem Halteelement und seiner Anbindung an den Plattenhauptkörper an.

Um eine einfache Anbindung des jeweiligen Haltefingers an den Plattenhauptkörper und dennoch eine gewisse Länge zwischen dem Halteelement und der Anwendung zu realisieren, kann die umlaufende Wand zumindest einen oberen Abschnitt eines oben offenen Sacklochs umschließen, an dessen Grund der Haltefinger an dem Plattenhauptkörper gelagert ist. Die Länge des Sacklochs entspricht dann der Länge des Haltefingers zwischen dem Halteelement und seiner Anbindung an den Plattenhauptkörper. Das Sackloch ist oben am Austritt des Haltefingers offen und Teil der Vertiefung, aber nicht Teil des Ringkanals.

Der jeweilige Halterfinger kann zum Beispiel stoffschlüssig, d. h. chemisch an den Grund des Sacklochs angebunden sein. Dies setzt jedoch ein unmittelbares Anformen oder ein problematisches Ankleben des Haltefingers an den Plattenhauptkörper am Grund des Sacklochs voraus.

In einer Ausführungsform der erfindungsgemäßen Lochplatte ist der oder jeder Haltefinger einstückig mit einer Bodenplatte ausgebildet, die kraftschlüssig an dem Grund des Sacklochs gelagert ist. Dazu kann das Sackloch nach unten konisch zulaufen, und die Bodenplatte kann mit leichtem Übermaß aus elastischem Material ausgebildet und in das Sackloch eingedrückt sein. Für ein kraftschlüssiges Halten der Bodenplatte am Grund des Sacklochs kann aber bereits eine günstige Materialkombination mit hoher Oberflächenspannung für in das Sackloch gelangende wässrige Flüssigkeiten ausreichen. Solange die gewünschte elastische Abstützung der Halteelemente erreicht wird, kann das Sackloch von der Bodenplatte oder einem anderen Montagekörper für die Haltefinger auch weitergehend und sogar vollständig ausgefüllt werden. Wie bereits angedeutet wurde, kann der oder jeder Haltefinger biegeelastisch sein. Dies schließt nicht aus, dass er zusätzlich mit der oben angesprochenen Bodenplatte aus einem biegeelastischen Material ausgebildet ist und damit auch elastisch an dem Plattenhauptkörper gelagert ist. Alternativ kann nur die Bodenplatte aus einem biegeelastischen Material ausgebildet sein, um eine Biegeelastizität der oder jeder Haltefinger zu erreichen, ohne dass das Material der oder jeder Haltefinger selbst biegeelastisch ist.

Alle auftretenden Elastizitäten, die sich auf die elastische Abstützung der mindestens zwei Halteelemente aneinander auswirken, können so aufeinander abgestimmt sein, dass eine horizontale Kraft von 1 mN zwischen den mindestens zwei Halteelementen an den beiden zugehörigen Haltefingern zu einer relativen horizontalen Auslenkung von freien Enden der beiden Haltefinger um 1 bis 50 % eines Ruheabstands der unbelasteten Haltefinger oder - beispielsweise im Fall einer Lochplatte zur Herstellung und Untersuchung von Ringen aus künstlichem Herzmuskelgewebe - von 10 bis 30% führt. Zugleich oder alternativ kann die horizontale Kraft von 1 mN zwischen den mindestens zwei Halteelementen an den beiden zugehörigen Haltefingern zu einer relativen horizontalen Auslenkung der freien Enden der beiden Haltefinger im Bereich von 20 bis 1.000 µm oder von 50 bis 500 µm führen. Es versteht sich, dass die Auslenkung keinen beliebigen Wert in dem genannten Bereich einnimmt, sondern mit der Kraft anwächst und bei der horizontalen Kraft von 1 mN den durch die Elastizität der Halterfinger und ihrer Anbindung definierten Wert annimmt.

Die hierfür notwendige Elastizität der Haltefinger lässt sich beispielsweise dadurch erreichen, dass der oder jeder Haltefinger einen Durchmesser von 0,5 bis 3 mm oder spezieller von 0,75 bis 2 mm aufweist und/oder dass der oder jeder Haltefinger eine Länge von 5 bis 10 mm vom Grund des Sacklochs bis zu dem an ihm ausgebildeten Halteelement und/oder eine Gesamtlänge von 10 bis 20 mm aufweist. Die Gesamtlänge der Haltefinger spielt insofern eine nicht unerhebliche Rolle, als dass die relative Auslenkung der Haltefinger im Bereich der daran ausgebildeten Halteelemente durch einen über das Halteelement hinaus nach oben überstehenden Zeigerbereich t vergrößert werden kann. Eine Kraft von x mN, die den Abstand der Halteelemente um y µm ändert, kann beispielsweise den Abstand von freien oberen Enden der Zeigerbereiche um 2y µm ändern und so zwischen den freien oberen Enden der Zeigerbereiche leichter erfasst werden. Zudem kann der oder jeder Haltefinger aufgrund seiner Gesamtlänge aus einer in die Vertiefung eingefüllten Nährlösung heraus ragen und seine Ausrichtung so besonders leicht optisch erfasst werden.

Als Material zur Ausbildung des oder jedes elastischen Haltefingers kann insbesondere solches mit einem Elastizitätsmodul im Bereich von 0,5 bis 50 MPa oder spezieller von 1 bis 10 MPa, d. h. von etwa 5 MPa verwendet werden. Auch hier gilt, dass der genannte Bereich kein Toleranzbereich ist, sondern das Material einen möglichst konstanten Elastizitätsmodul innerhalb des Bereichs aufweist.

Auch wenn der oder jeder Haltefinger mit seinem freien Ende über das an ihm ausgebildete Halteelement hinaussteht, endet er vorzugsweise unterhalb der Oberseite des Plattenhauptkörpers. Damit ist eine Gefahr von Beschädigungen der Haltefinger beispielsweise durch Stapeln erfindungsgemäßer Lochplatten oder anderweitiges Handhaben der erfindungsgemäßen Lochplatte reduziert.

Vorzugsweise ist der oder jeder Haltefinger aus einem optisch gegenüber dem Plattenhauptkörper kontrastierenden Material ausgebildet. Hiermit ist beispielsweise gemeint, dass der Plattenhauptkörper aus weißem, anderem hellem oder transparentem Material ausgebildet ist, während der oder jeder Haltefinger aus schwarzem oder dunklem und/oder nichttransparentem Material ausgebildet ist. Die Haltefinger kontrastieren dann in der Regel auch gut gegenüber einer in die Vertiefung eingebrachten Nährlösung. Der oder jeder Haltefinger kann alternativ oder zusätzlich auch aus einem im Gegensatz zu dem Plattenhauptkörperfluoreszierenden Material ausgebildet sein, um den optischen Kontrast bei Beleuchtung mit Anregungslicht für das fluoreszierende Material herbeizuführen.

In einer Ausführungsform der erfindungsgemäßen Lochplatte sind mindestens zwei der Halteelemente an Haltefingern ausgebildet, die jeweils zumindest einen sich bis in das Halteelement erstreckenden elektrisch leitfähigen Teilbereich aufweisen, wobei die elektrisch leitfähigen Teilbereiche der mindestens zwei Halterfinger von der Unterseite des Plattenhauptkörpers her elektrisch kontaktierbar sind. Unter Nutzung dieser elektrischen Kontaktierbarkeit können an den Halteelementen anliegende Ringe elektrisch angeregt oder gereizt werden. Dies gilt sowohl für Ringe aus künstlichem Muskelgewebe wie Herzmuskel- oder Skelettmuskelgewebe oder für Ringe aus künstlichem Nervengewebe als auch für Ringe aus elektrorheologischem Material.

In einer anderen Ausführungsform der erfindungsgemäßen Lochplatte ist eines der Halteelemente an zumindest einem Kontaktpunkt mit dem Gewebe elektrisch leitend, um eine Elektrode auszubilden. Dieses Halteelement kann, muss aber nicht das mindestens eine elastisch an dem Plattenhauptkörper abgestützte Halteelement sein. Eine Gegenelektrode ist an anderer Stelle in der Vertiefung vorgesehen, so dass sie das eingefüllte Nährmedium kontaktiert. Die an dem Halteelement ausgebildete Elektrode und die Gegenelektrode können von außerhalb der Vertiefung elektrisch kontaktierbar sein.

Zur Ausbildung des jeweiligen Halteelements kann der oder jeder Haltefinger eine nach oben an die den Ringkanal an seinem Umfang begrenzte umlaufende Wand anschließende horizontale Einbuchtung oder eine etwas darüber liegende Ausbuchtung aufweisen. Von dieser Ein- oder Ausbuchtung wird der jeweilige Ring in Position gehalten, wenn er sich von der umlaufenden Wand ablöst. Es stellt sich jedoch heraus, dass eine solche Ein- oder Ausbuchtung zur Ausbildung eines geometrisch definierten Halteelements nicht unbedingt erforderlich ist. Der jeweilige Ring behält nach dem Ablösen von der umlaufenden Wand seine Position auf jedem Haltefinger direkt über der Oberkante der umlaufenden Wand auch dann bei, wenn die Haltefinger nach oben glatt durchlaufen.

Der horizontale Außenumfang der umlaufenden Wand kann insbesondere aus zwei zueinander parallelen Geradenabschnitten und zwei die Enden der beiden Geradenabschnitte miteinander verbindenden Halbkreisbögen zusammengesetzt sein. Hieraus resultiert ein parallel zu den Geradenabschnitten gestreckter Ring, der daran angepasst ist, zwischen zwei Halteelementen gespannt zu werden, die entsprechend oberhalb in den Halbkreisbögen des horizontalen Außenumfangs oberhalb der Oberkante der umlaufenden Wand anzuordnen sind.

In dieser Ausführungsform der erfindungsgemäßen Lochplatte kann sowohl der Abstand der Geradenabschnitte als auch ein Radius der Halbkreisbögen von unten nach oben abnehmen, so dass die Wand überall einen Neigungswinkel gegenüber der Vertikalen aufweist. Dieser Neigungswinkel kann zwischen 15 ° bis 65 ° oder spezieller zwischen 35 ° und 55 °, d. h. etwa 45 ° betragen. Dabei muss der Neigungswinkel nicht über den gesamten Außenumfang der umlaufenden Wand gleich sein. Beispielsweise kann der Neigungswinkel im Bereich der Geradenabschnitte größer sein als im Bereich der Halbkreisbögen, weil dort die Spannung des sich bildenden Rings und entsprechend die durch die Neigung der Wand nach oben gerichtete Kraft größer sein kann.

Der Ringkanal kann an seinem Außenumfang durch eine umlaufende Wand begrenzt sein, die zumindest in einem Abschnitt einen Neigungswinkel gegenüber der Vertikalen nach außen im Bereich von 15 bis 65 °oder spezieller von 35 bis 55 °, d. h. von etwa 45 ° aufweist. So wird eine Einlauffläche für das sanfte Einlaufenlassen einer Suspension in den Ringkanal ausgebildet.

Der Ringkanal kann ein Volumen von 50 bis 1.500 µl oder spezieller von 100 bis 250 µl aufweisen. Diese Dimensionierung des Ringkanals ist für die Ausbildung von Ringen mit einem Innenumfang von 6 bis 20 mm gut geeignet.

Für eine elektrische Reizung des Materials des Rings durch ein elektrisches Feld können in Richtung des horizontalen Abstands der mindestens zwei Halteelemente zwei Elektrodenfreiplätze am Außenumfang der mindestens einen Vertiefung ausgebildet sein. Hier können Elektroden von oben in die Vertiefung eintauchen, die typischerweise nicht Bestandteil der Lochplatte selbst sind. Für die Reizung des Materials des Rings durch ein magnetisches Feld kann die gesamte Lochplatte in eine oder zwischen zwei Ringspulen eingebracht werden.

Regelmäßig verlaufen ein Grund des Ringkanals und die Oberkante der umlaufenden Wand bei dem der erfindungsgemäßen Lochplatte horizontal, und die mindestens eine Vertiefung ist bis auf ihre Öffnung an der Oberseite des Plattenkörpers geschlossen.

Wenn die erfindungsgemäße Lochplatte zum Herstellen und Untersuchen von künstlichem Gewebe verwendet werden soll, ist die mindestens eine Vertiefung vollständig mit biokompatiblem Material auszukleiden, wozu der Plattengrundkörper und die daran angesetzten Haltefinger insgesamt aus biokompatiblem Material ausgebildet sein können. Auch wenn keine Ringe aus biologischem Material ausgebildet werden, ist es bevorzugt, wenn die umlaufende Wand, die den Ringkanal an seinem Innenumfang begrenzt, aus einem hydrophoben Material ausgebildet ist. Weiterhin ist es bevorzugt, wenn dieses Material glatt ist, d. h. eine geringer Oberflächenrauheit aufweist. Damit wird die Haftreibung zwischen der umlaufenden Wand und dem Ring reduziert.

Für die Herstellung einer Vielzahl von Ringen und umfangreichen Untersuchungen kann die erfindungsgemäße Lochplatte mit mehreren Vertiefungen in zwei zueinander orthogonalen horizontalen Richtungen versehen sein.

Bei einer erfindungsgemäßen Verwendung der erfindungsgemäßen Lochplatte wird eine Suspension von Bindegewebszellen und weiteren Zellen in den Ringkanal gegeben. Die Vertiefung wird bis über die Oberkante der umlaufenden Wand und vorzugsweise bis über die Halteelemente mit einer Nährlösung aufgefüllt. Bei der Reifung des Gewebes erhöht sich seine Spannung und es steigt die umlaufende Wand nach oben, bis es von den Halteelementen gehalten wird. Dann auftretende Kontraktionen des Rings, die bei Herzmuskelgewebe spontan sind und bei Skelettmuskelgewebe beispielsweise durch elektrische Reizung hervorgerufen werden können, werden anhand von Bewegungen eines freien oberen Endes des mindestens einen elastisch abgestützten Haltelements oder jedes Halteelements oder eines daran nach oben anschließenden Zeigerbereichs mit einer Kamera erfasst.

Die Suspension kann insbesondere eine wässrige Suspension eines Hydrogels und von Muskelzellen, speziell von Herzmuskel- oder Skelettmuskelzellen, sein.

Die weiteren Zellen können auch Hepatozyten oder Neuronen, d. h. Nervenzellen, sein.

Das Auffüllen der Vertiefung mit der Nährlösung kann nach einer vorgegebenen Wartezeit erfolgen, die so gewählt wird, dass bei Zugabe der Nährlösung ein Ausschwemmen von Komponenten des Hydrogels zu vermieden wird.

Die Vertiefung kann bis über die Halteelemente mit der Nährlösung aufgefüllt wird, wobei ein freies oberes Ende des oder jedes Haltefingers über die Nährlösung überstehen kann, um das Erfassen von Bewegungen des freien oberen Endes des oder jedes Haltefingers mit einer Kamera zu vereinfachen. Grundsätzlich kann die Nährlösung die mit der Kamera beobachteten freien Enden aber auch überdecken.

Nicht nur zum elektrischen Anregen von künstlichem Skelettmuskelgewebe sondern auch zum elektrischen Anregen von künstlichem Herzmuskelgewebe oder elektrorheologischem Material kann eine elektrische Spannung mit veränderlicher Amplitude, beispielsweise in Form einzelner Spannungspulse oder in Form einer uni- oder bipolaren Rechteckspannung, zwischen den leitfähigen Teilbereichen der Haltefinger oder zwischen Elektroden an den Elektrodenfreiplätzen der erfindungsgemäßen Lochplatte angelegt werden, während die Bewegungen des freien oberen Endes des oder jedes Haltefingers mit der Kamera erfasst werden. Konkret kann die erfasste Bewegung mit der Zusammensetzung der Suspension und/oder der Frequenz und der Amplitude der elektrischen Spannung und/oder der Zusammensetzung und Temperatur der Nährlösung und/oder Zusätzen zu der Nährlösung oder dergleichen korreliert werden. Beispielsweise können der Nährlösung verschiedene pharmakologisch oder toxikologisch aktive Substanzen oder Wirkstoffe zugesetzt werden, und es kann dann geprüft werden, bei welchen aktiven Substanzen oder Wirkstoffen bzw. welcher Konzentration der aktiven Substanzen oder Wirkstoffe ein quantifizierbarer Effekt zum Beispiel auf die Kontraktilität in Bezug auf Auslenkung, spontaner Schlagfrequenz, Grundspannung, Geschwindigkeit des Zusammenziehens bzw. des Entspannens erkennbar ist.

Die elektrische Anregung des künstlichen Gewebes kann auch ohne Beobachten mit der Kamera erfolgen, um beispielsweise die Reifung des Gewebes zu fördern oder gezielt zu beeinflussen.

Falls die in die Vertiefung eingebrachte Nährlösung farbig ist, wird sie farblich vorzugsweise so auf den oder jeden Haltefinger abgestimmt, dass der oder jeder Haltefinger optisch gegenüber der Nährlösung kontrastiert. Beispielsweise kann die Nährlösung aufgrund eines pH-Indikators farbig sein. Es versteht sich, das die farbliche Abstimmung der Nährlösung auf den oder jeden Haltefinger impliziert, das für Nährlösungen bestimmter Farbe der oder jeder Haltefinger in einer dazu kontrastierenden Farbe vorgesehen werden kann/können.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Element die Rede ist, ist dies so zu verstehen, dass genau ein Element, zwei Elemente oder mehr Elemente vorhanden sind. Diese Merkmale können durch andere Merkmale ergänzt werden oder die einzigen Merkmale sein, aus denen das jeweilige Erzeugnis besteht.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt eine erfindungsgemäße Lochplatte mit mehreren Vertiefungen in ihrer Oberseite in einer Ansicht von oben.
- **Fig. 2**: ist eine perspektivische Ansicht einer der Vertiefungen der Lochplatte gemäß Fig. 1.
- **Fig. 3**: zeigt einen vertikalen Längsschnitt durch die Vertiefung gemäß Fig.2.
- **Fig. 4**: zeigt einen vertikalen Querschnitt durch die Vertiefung gemäß Fig. 2.
- **Fig. 5**: zeigt einen vertikalen Längsschnitt durch eine etwas andere Ausführungsform der Vertiefung gemäß Fig. 2 bei einer erfindungsgemäßen Verwendung der erfindungsgemäßen Lochplatte; und
- **Fig. 6**: ist ein Blockdiagramm des erfindungsgemäßen Verfahrens.

### FIGURENBESCHREIBUNG

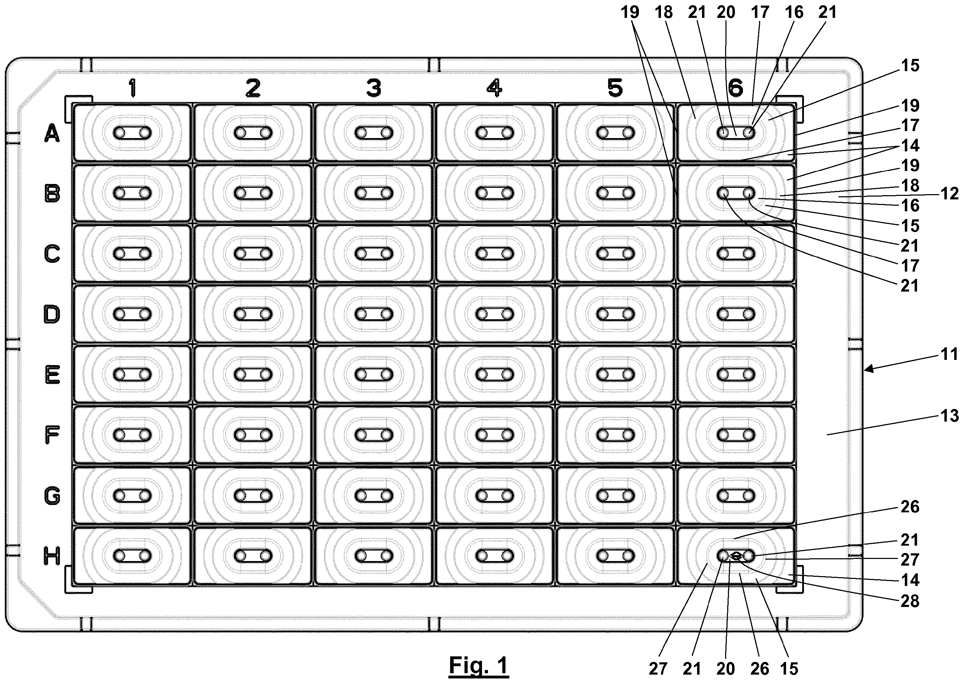

Die in **Fig. 1** dargestellte Lochplatte 11 weist einen Grundkörper 12 auf, der eine grundsätzlich ebene Oberseite 3 der Lochplatte 11 ausbildet. In der Oberseite 13 ist eine Vielzahl von Vertiefungen 14 ausgebildet, die jeweils rechteckige horizontale Gesamtabmessungen aufweisen. Die Vertiefungen 14 sind in acht Zeilen A bis H und sechs Spalten 1 bis 6 angeordnet. Am Grund jeder Vertiefung 14 ist ein Ringkanal 15 ausgebildet, der an seinem Innenumfang durch eine umlaufende Wand 16 begrenzt ist. An seinem Außenumfang ist der Ringkanal 15 umlaufend abschnittweise durch Längswände 17, die die jeweilige Vertiefung an ihrer Langseite begrenzen, und abschnittweise durch Wände 18 begrenzt, die nach oben in Querwände 19 übergehen, welche die jeweilige Vertiefung 14 an ihrer Schmalseite begrenzen. Innerhalb der umlaufenden Wand 16 ist in jeder Vertiefung 14 ein Sackloch 20 ausgebildet aus dem zwei Haltefinger 21 nach oben vorstehen. Der horizontale Außenumfang der umlaufenden Wand - und entsprechend auch der Ringkanal 15 am Grund jeder Vertiefung 14 - ist aus zwei parallel zueinander verlaufenden Geradenabschnitten 26 und zwei die freien Enden der Geradenabschnitte 26 miteinander verbindenden Halbkreisbögen 27 zusammengesetzt. Das Sackloch 20 ist in der Horizontalen in der Längsrichtung der jeweiligen Vertiefung 14 gestreckt und erstreckt sich dabei über die geraden Abschnitte bis in die Halbkreisbögen. Die Haltefinger 21 sind an den beiden Enden des langgestreckten Sacklochs 20 im Wesentlichen vertikal ausgerichtet und in einem freien horizontalen Abstand 28 zueinander angeordnet.

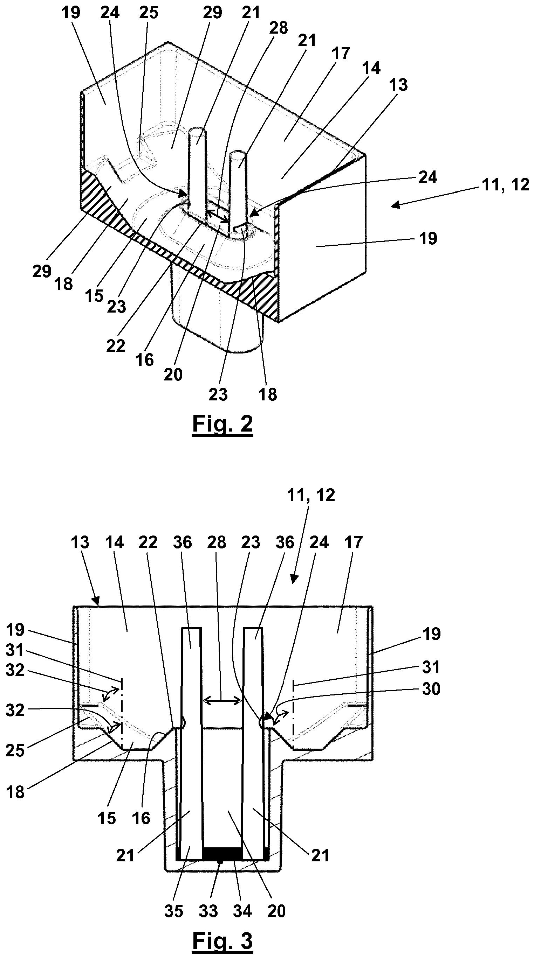

**Fig.** 2 zeigt eine der Vertiefungen 14 der Lochplatte 11 in einer perspektivischen Ansicht, wobei eine ihrer Längswände 17 entfernt ist, um den Blick in das Innere der Vertiefung 14 freizugeben. Dadurch ist zu sehen, dass oberhalb einer Oberkante 22 der umlaufenden Wand 16 Einbuchtungen 23 in den Haltefingern 21 vorgesehen sind, um Halteelemente 24 auszubilden. Weiterhin sind an den Schmalseiten der Vertiefung 14 vor den Wänden 19 Elektrodenfreiplätze 25 auf Höhe der Halteelemente 24 freigelassen. Fig. 2 lässt deutlich werden, dass der horizontale Außenumfang der umlaufende Wand 16 einen von unten nach oben abnimmt und dass die Halteelemente 24 so an die Oberkante 22 der umlaufenden Wand 16 angrenzen, dass sie die umlaufende Wand 16 bis auf den freien Abstand 28 der Haltefinger 21 nach oben fortsetzen. Neben den Elektrodenfreiplätzen steigen die nach außen geneigten äußeren Wände 18 des Ringkanals 15 bereichsweise an und bilden so Einlaufflächen 29, über die der Ringkanal 15 kontrolliert befüllt werden kann.

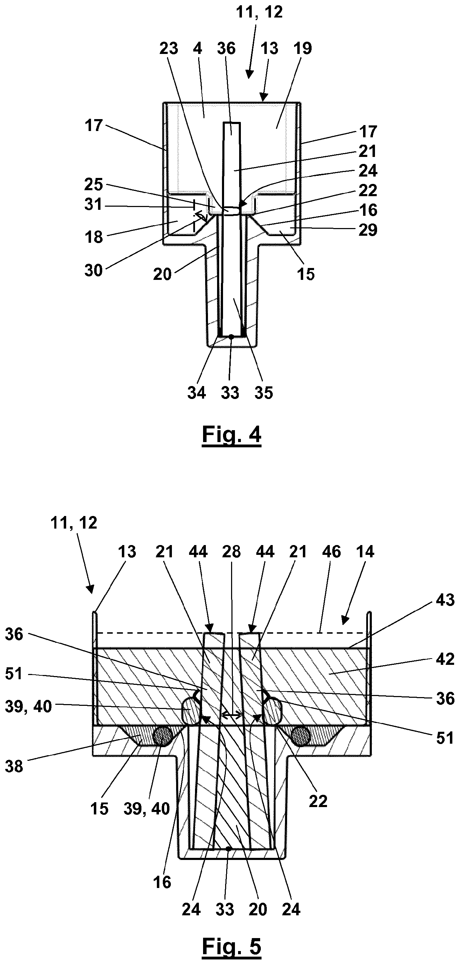

In dem Längsschnitt gemäß **Fig. 3** und dem Querschnitt gemäß **Fig. 4** ist ein Neigungswinkel 30 der umlaufenden Wand 16 zur Vertikalen 31 hervorgehoben, der hier jeweils 45 ° beträgt, aber grundsätzlich auch kleiner oder größer sein kann. Ein Neigungswinkel 32 der Wand 18 gegenüber der Vertikalen 31 beträgt hier ebenfalls etwa 45 °, kann aber auch kleiner oder größer sein. Die Befestigung der Haltefinger 21 am Grund 33 des Sacklochs 20 ist über eine Bodenplatte 34 bewirkt, die einstückig mit den Haltefingern 21 aus einem elastischen Material 35 ausgebildet ist. Die Bodenplatte 34 kann kraftschlüssig in das Sackloch 20 eingesetzt sein. Häufig reichen hierfür gut aufeinander abgestimmte Oberflächenspannungen aus, um die Bodenplatte 34 am Grund 33 des Sacklochs 20 zu halten und so eine definierte Grundausrichtung der Haltefinger 21 in der Vertiefung 14 zu bewirken. Die Haltefinger 21 können sich nach oben leicht verjüngen, um aus einem Formwerkzeug leicht entformbar zu sein. Entsprechend kann sich auch der freie Querschnitt der Vertiefung 14 an allen Stellen, wie innerhalb des Sacklochs 20, nach oben leicht erweitern, um ein Entformen des Grundkörpers 12 aus einem Formwerkzeug zu erleichtern. Die Haltefinger 21 stehen mit Zeigerbereichen 36 nach oben über die an ihnen ausgebildeten Halteelemente 24 über, enden aber unterhalb der Oberseite 13 des Plattenhauptkörpers 12.

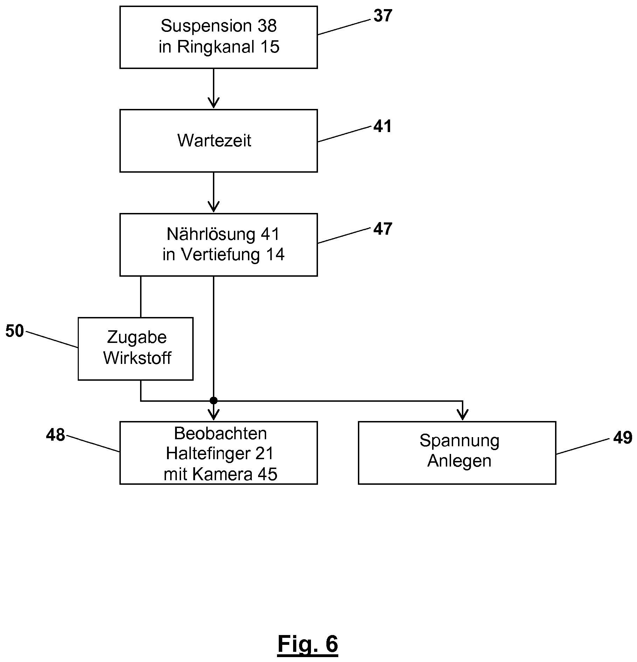

**Fig. 5** illustriert anhand einer Vertiefung 14 und **Fig. 6** illustriert anhand eines Blockdiagramms die erfindungsgemäße Verwendung der erfindungsgemäßen Lochplatte 11. In einem ersten Schritt 37 wird der Ringkanal 15 mit einer Suspension 38 beispielsweise eines Hydrogels und von Herzmuskelzellen in einer wässrigen Lösung befüllt. In einem Schritt 41 verfestigt sich das Hydrogel mit den Herzzellen binnen einer Wartezeit von idealtypisch 30 min bis wenigen Stunden. Dann kann die Vertiefung 14 in einem Schritt 47 bis auf ein Niveau 43 mit einer Nährlösung 42 aufgefüllt werden. In der Nährlösung 42 bildet sich typischerweise binnen 24 bis 72 h ein Ring 39 aus künstlichem Herzmuskelgewebe 40. Der Ring 39 kontrahiert und wandert so die umlaufende Wand 16 hinauf. Das Niveau 43 der Nährlösung wird so gewählt, dass der Ring 39 vollständig in der Nährlösung verbleibt wenn er die Oberkante 22 der umlaufenden Wand 16 übersteigt und dann an den Halteelementen 24 an den Haltefingern 21 anliegt. Die Halteelemente 24 weisen an ihrem oberen Ende horizontale Ausbuchtungen 51 der Haltefinger 21 auf. Sie sind über die Haltefinger 21 elastisch aneinander abgestützt und wirken so als Stretcher für den an ihnen anliegenden Ring 39. Bei Ausbildung des Rings 39 aus künstlichem Herzmuskelgewebe beginnt der Ring 39 selbsttätig mit periodischen Kontraktionen. Die Frequenz dieser Kontraktionen kann in einem Schritt 49 durch ein externes elektrisches Feld oder direkte elektrische Reizung des Rings 39 mit einer elektrischen Spannung veränderlicher Amplitude, beispielsweise in Form einzelner Spannungspulse oder in Form einer uni- oder bipolaren Rechteckspannung, vorgegeben werden. Bei Ausbildung des Rings 39 beispielsweise aus künstlichem Skelettmuskelgewebe oder auch aus elektrorheologischem Material werden Kontraktionen überhaupt nur durch elektrische Felder oder direkt angelegte elektrische Spannungen hervorgerufen.

Erfassbar sind die Kontraktionen in jedem Fall in einem Schritt 48 durch Beobachten der freien Enden 44 mit Hilfe einer Kamera 45, deren Fokusebene 46 so angeordnet ist, dass sie genau die freien Enden 44 scharf abbildet. So werden mit der Kamera 45 Änderungen des freien Abstands 28 nach zeitlichem Verlauf und Größer quantitativ erfasst. Günstige optische Verhältnisse für das Beobachten der Bewegungen der freien Enden 44 mit der Kamera 45 sind dadurch gegeben, dass das Niveau 43 der Nährlösung 42 unterhalb der freien Enden 44 der Zeigerbereiche 36 der Haltefinger 21 liegt. Die aus der Nährlösung 42 heraus ragenden freien Enden 44 sorgen für einen höheren Kontrast in den Bildern der Kamera, als wenn sie von der Nährlösung 42 überdeckt wären, was bei der erfindungsgemäßen Verwendung der erfindungsgemäßen Lochplatte 11 aber grundsätzlich auch möglich ist.

Fig. 6 illustriert dass der Schritt 49 des Anlegens der elektrischen Spannung parallel zu dem Schritt 48 des Beobachtens der Haltefinger 21 mit der Kamera 45 erfolgt. Bei oder nach dem Schritt 47 des Zugebens der Nährlösung 42 in die Vertiefung 14 kann ein weiterer optionaler Schritt 50 erfolgen, in dem der Nährlösung 42 ein Wirkstoff zugesetzt wird. Dieser Schritt 50 kann vor oder während der Schritte 48 und 49 erfolgen, wobei der Schritt 49 optional ist. So können die Auswirkungen dieses Wirkstoffes auf das künstliche Herzmuskelgewebe 40 untersucht werden, beispielsweise dahingehend, wie sich die Kontraktilität, gemessen als dynamische Änderung des freien Abstands 28 der Haltefinger 21, verändert.

In Fig. 5 sind die beiden Haltefinger 21 nicht durch eine Bodenplatte miteinander verbunden, sondern einzeln stoffschlüssig an dem Grund 33 des Sacklochs 20 angebunden. Dies kann beispielsweise beim Drucken der Lochplatte in einem 3D-Drucker erfolgen oder durch Verkleben bzw. Verschmelzen der Haltefinger mit der Lochplatte.

### BEZUGSZEICHENLISTE

- A bis H: Zeile
- 1 bis 6: Spalte
- 11: Lochplatte
- 12: Plattenhauptkörper
- 13: Oberseite
- 14: Vertiefung
- 15: Ringkanal
- 16: umlaufende Wand
- 17: Längswand
- 18: Wand
- 19: Querwand
- 20: Sackloch
- 21: Haltefinger
- 22: Oberkante
- 23: Einbuchtung
- 24: Halteelement
- 25: Elektrodenfreiplatz
- 26: Geradenabschnitt
- 27: Halbkreisbogen
- 28: freier Abstand
- 29: Ablauffläche
- 30: Neigungswinkel
- 31: Vertikale
- 32: Neigungswinkel
- 33: Grund des Sacklochs
- 34: Bodenplatte
- 35: Material
- 36: Zeigerbereich
- 37: Schritt
- 38: Suspension
- 39: Ring
- 40: künstliches Herzmuskelgewebe
- 41: Schritt
- 42: Nährlösung
- 43: Niveau
- 44: freies Ende
- 45: Kamera
- 46: Fokusebene
- 47: Schritt
- 48: Schritt
- 49: Schritt
- 50: Schritt
- 51: Ausbuchtung

## Patentansprüche

1. Lochplatte (11) mit einem Plattenhauptkörper (12) und mit mindestens einer Vertiefung (14) in einer Oberseite (13) des Plattenhauptkörpers (12),
- wobei in der mindestens einen Vertiefung (14) ein oben offener Ringkanal (15) ausgebildet ist, der an seinem Innenumfang durch eine geschlossen umlaufende Wand (16) begrenzt ist,
**dadurch gekennzeichnet,**
- **dass** ein horizontaler Außenumfang der umlaufenden Wand (16) von unten nach oben bis an eine Oberkante (22) der umlaufenden Wand (16) abnimmt und
- **dass** innerhalb des horizontalen Außenumfangs der umlaufenden Wand (16) an ihrer Oberkante (22) mindestens zwei Halteelemente (24) nach oben an die Oberkante (22) der umlaufenden Wand (16) anschließen,
- wobei die mindestens zwei Halteelemente (24) einen freien horizontalen Abstand (28) voneinander aufweisen und
- wobei mindestens eines der mindestens zwei Haltelemente (24) in horizontaler Richtung elastisch an dem Plattenhauptkörper (12) abgestützt ist.

2. Lochplatte (11) nach Anspruch 1, **dadurch gekennzeichnet, dass** jedes der mindestens zwei Halteelemente (24) in der mindestens einen Vertiefung (14) an dem Plattenhauptkörper (12) abgestützt ist.

3. Lochplatte (11) nach einem der vorhergehenden Ansprüche, **dadurch gekennzeichnet, dass** das mindestens eine der Haltelemente (24) oder jedes Halteelement (24) jeweils Teil eines sich längs der Vertikalen (31) erstreckenden Haltefingers (21) ist.

4. Lochplatte (11) nach Anspruch 3, **dadurch gekennzeichnet, dass** die umlaufende Wand (16) zumindest einen oberen Abschnitt eines oben offenes Sacklochs (20) umschließt, an dessen Grund (33) der oder jeder Haltefinger (21) an dem Plattenhauptkörper (12) gelagert ist, wobei optional der oder jeder Haltefinger (21) einstückig mit einer Bodenplatte (34) ausgebildet ist, die kraftschlüssig an dem Grund (33) des Sacklochs (20) gelagert ist.

5. Lochplatte (11) nach einem derAnsprüche 3 und 4, **dadurch gekennzeichnet,**
- **dass** der oder jeder Haltefinger (21) biegeelastisch ist und/oder
- **dass** die mindestens zwei Halteelemente (24) an zwei Haltefingern (21) so elastisch aneinander abgestützt sind, dass eine horizontale Kraft von 1 mN zwischen den mindestens zwei Halteelementen (24) zu einer relativen horizontalen Auslenkung von freien Enden (44) der beiden Haltefinger (21) im Bereich von 20 bis 1.000 µm oder von 50 bis 500 µm und/oder im Bereich von 1 bis 50 % oder von 10 bis 30 % eines Ruheabstands der Haltefinger (21) führt, und/oder
- **dass** der oder jeder Haltefinger (21) einen Durchmesser von 0,5 bis 3,0 mm oder von 0,75 bis 2,0 mm aufweist und/oder dass der oder jeder Haltefinger (21) eine Länge von 5 bis 10 mm von dem Grund (33) des Sacklochs (20) bis zu dem an ihm ausgebildeten Halteelement (24) und/oder eine Gesamtlänge bis zu seinem freien Ende (44) von 10 bis 20 mm aufweist und/oder
- **dass** das Material (35) des oder jedes Haltefingers (21) einen Elastizitätsmodul im Bereich von im Bereich von 0,5 bis 50 MPa oder von 1 bis 10 MPa aufweist.

6. Lochplatte (11) nach einem der Ansprüche 3 bis 5, **dadurch gekennzeichnet,**
- **dass** sich der oder jeder Haltefinger (21) mit einem Zeigerbereich (36) bis über das Halteelement (24) hinaus nach oben erstreckt und/oder
- **dass** der oder jeder Haltefinger (21) unterhalb der Oberseite (13) des Plattenhauptkörpers (12) endet und/oder
- **dass** der oder jeder Haltefinger (21) aus einem optisch gegenüber dem Plattenhauptkörper (12) kontrastierenden und/oder einem fluoreszierenden Material (35) ausgebildet ist.

7. Lochplatte (11) nach einem der Ansprüche 3 bis 6, **dadurch gekennzeichnet, dass** die mindestens zwei Halteelemente (24) an zwei Haltefingern (21) ausgebildet sind, die jeweils zumindest einen sich bis in das Halteelement erstreckenden elektrisch leitfähigen Teilbereich aufweisen, und dass die elektrisch leitfähigen Teilbereiche der mindestens zwei Haltefinger (21) von der Unterseite des Plattenhauptkörpers (12) her elektrisch kontaktierbar sind.

8. Lochplatte (11) nach einem der Ansprüche 3 bis 7, **dadurch gekennzeichnet, dass** der oder jeder Haltefinger (21) im Bereich des Halteelements (24) eine horizontale Ein- oder Ausbuchtung (23, 51) aufweist.

9. Lochplatte (11) nach einem der vorhergehenden Ansprüche, **dadurch gekennzeichnet, dass** der horizontale Außenumfang der umlaufenden Wand (16) aus zwei zueinander parallelen Geradenabschnitten (26) und zwei die Enden der beiden Geradenabschnitte (26) miteinander verbindenden Halbkreisbögen (27) zusammengesetzt ist, wobei optional der Abstand der Geradenabschnitte (26) und/oder ein Radius der Halbkreisbögen (27) von unten nach oben abnimmt.

10. Lochplatte (11) nach einem der vorhergehenden Ansprüche, **dadurch gekennzeichnet,**
- **dass** ein Neigungswinkel (30) der umlaufenden Wand (16) gegenüber der Vertikalen (31) 15 ° bis 65 °oder 35 ° bis 55 "beträgt und/oder
- **dass** der Ringkanal (15) an seinem Außenumfang durch Wände (17, 18) umlaufend begrenzt ist, die zumindest teilweise einen Neigungswinkel (32) gegenüber der Vertikalen (31) nach außen im Bereich von 15 ° bis 65 °oder von 35 ° bis 55 °aufweist.

11. Lochplatte (11) nach einem der vorhergehenden Ansprüche, **dadurch gekennzeichnet, dass** der Ringkanal (15) ein Volumen von 50 bis 1.500 µl oder von 100 bis 250 µl aufweist.

12. Lochplatte (11) nach einem der vorhergehenden Ansprüche, **dadurch gekennzeichnet, dass** in Richtung des horizontalen Abstands (28) der mindestens zwei Halteelemente (24) zwei Elektrodenfreiplätze (25) am Außenumfang der mindestens einen Vertiefung (14) ausgebildet sind.

13. Lochplatte (11) nach einem der vorhergehenden Ansprüche, **dadurch gekennzeichnet, dass** ein Grund des Ringkanals (15) und die Oberkante (22) der umlaufenden Wand (16) horizontal verlaufen und dass die mindestens eine Vertiefung (14) bis auf ihre Öffnung an der Oberseite des Plattenhauptkörpers (12) geschlossen ist.

14. Lochplatte (11) nach einem der vorhergehenden Ansprüche, **dadurch gekennzeichnet, dass** die mindestens eine Vertiefung (14) vollständig mit biokompatiblem Materialien ausgekleidet ist und/oder dass die umlaufende Wand (16) aus einem glatten und/oder hydrophobem Material ausgebildet ist.

15. Lochplatte (11) nach einem der vorhergehenden Ansprüche, **dadurch gekennzeichnet, dass** in der Lochplatte (11) mehrere Vertiefungen (14) in zwei horizontalen Richtungen nebeneinander angeordnet sind.

16. Verwendung einer Lochplatte (11) nach einem der vorhergehenden Ansprüche, **dadurch gekennzeichnet, dass** eine Suspension (38) von Bindegewebszellen und weiteren Zellen in den Ringkanal (15) gegeben wird, dass die Vertiefung (14) bis über die Haltelemente (24) mit einer Nährlösung (42) aufgefüllt wird und dass Bewegungen eines freien oberen Endes (44) des mindestens einen der Haltelemente (24) oder jedes Halteelements (24) oder eines daran nach oben anschließenden Zeigerbereichs (36) mit einer Kamera (45) erfasst werden.

17. Verwendung nach Anspruch 16, **dadurch gekennzeichnet,**
- **dass** das freie obere Ende (44) des Zeigerbereichs (36) über die Nährlösung (42) übersteht und/oder
- **dass** die Nährlösung (42) farblich so auf den oder jeden Haltefinger (21) abgestimmt wird, dass der oder jeder Haltefinger (21) optisch gegenüber der Nährlösung (42) kontrastiert.

18. Verwendung nach Anspruch 16 oder 17, **dadurch gekennzeichnet, dass** die weiteren Zellen aus Muskelzellen, Herzmuskelzellen oder Skelettmuskelzellen ausgewählt werden.

19. Verwendung nach einem der Ansprüche 16 bis 18, **dadurch gekennzeichnet, dass** die Suspension weiterhin ein Hydrogel umfasst.

20. Verwendung nach einem der Ansprüche 16 bis 19, **dadurch gekennzeichnet, dass** eine elektrische Spannung veränderlicher Amplitude zwischen Elektroden an den Halteelementen (24) und/oder in der Nährlösung (42) angelegt wird.

## Claims

1. Well plate (11) comprising a plate main body (12) and at least one cavity (14) in an upper side (13) of the plate main body (12),
- wherein an upwardly open annular channel (15) is formed in the at least one cavity (14), the annular channel being delimited at an inner circumference thereof by a closed circumferential wall (16),
**characterized in**
- **that** a horizontal outer circumference of the circumferential wall (16) decreases from bottom to top up to an upper edge (22) of the circumferential wall (16), and
- **that**, within the horizontal circumference of the circumferential wall (16), at its upper edge (22), at least two retaining elements (24) connect upwardly to the upper edge (22) of the circumferential wall (16),
- wherein the at least two retaining elements (24) are at a free horizontal distance (28) to one another, and
- wherein at least one of the at least two retaining elements (24) is elastically supported at the plate main body (12) in horizontal direction.

2. Well plate (11) of claim 1, **characterized in that** each of the at least two retaining elements (24) is supported at the plate main body (12) in the at least one cavity (14).

3. Well plate (11) of any of the preceding claims, **characterized in that** the at least one of the retaining elements (24) or each retaining element (24) is a part of a retaining finger (21) extending along the vertical direction (31).

4. Well plate (11) of claim 3, **characterized in that** the circumferential wall (16) encloses at least an upper section of an upwardly open blind hole (20), at the bottom (33) of which the or each retaining finger (21) is supported at the plate main body (12), wherein, optionally, the or each retaining finger (21) is made in one part with a base plate (34) which is force fitted at the bottom (33) of the blind hole (20).

5. Well plate (11) of any of the claims 3 and 4, **characterized in**
- **that** the or each retaining finger (21) is bending elastic, and/or
- **that** the at least two retaining elements (24) provided at two retaining fingers (21) are elastically supported at one another in such a way that a horizontal force of 1 mN between the at least two retaining elements (24) results in a relative horizontal deflection of free ends (24) of the two retaining fingers (21) in a range from 20 to 1.000 µm or from 50 to 500 µm and/or in a range from 1 to 50 % or from 10 to 30 % of a resting distance of the retaining fingers (21), and/or
- **that** the or each retaining finger (21) has a diameter of 0.5 to 3.0 mm or of 0.75 to 2.0 mm and/or that the or each retaining finger (21) has a length of 5 to 10 mm from the bottom (33) of the blind hole (20) to the retaining element (24) formed at the holding finger (21) and/or an overall length up to its free end (44) of 10 to 20 mm, and/or
- **that** the material (35) of the or each retaining finger (21) has a modulus of elasticity in a range from 0.5 to 50 MPa or in a range from 1 to 10 MPa.

6. Well plate (11) of any of the claims 3 to 5, **characterized in**
- **that** the or each retaining finger (21) extends upwardly beyond the retaining element (24) with a pointer area (36), and/or
- **that** the or each retaining finger (21) ends below the upper side (13) of the plate main body (12), and/or
- **that** the or each retaining finger (21) is made of a material (35) which is optically contrastive with regard to the plate main body (12) and/or fluorescent.

7. Well plate (11) of any of the claims 3 to 6, **characterized in that** the at least two retaining elements (24) are formed at two retaining fingers (21) which each comprise at least one electrically conductive partial area extending up into the retaining element, and that the electrically conductive partial areas of the at least two retaining fingers (21) are electrically contactable from the bottom side of the plate main body (12).

8. Well plate (11) of any of the claims 3 to 7, **characterized in that** the or each retaining finger (21) has a horizontal notch (23) or bulge (51) in the area of the retaining element (24).

9. Well plate (11) of any of the preceding claims, **characterized in that** the horizontal circumference of the circumferential wall (16) is made up of two straight sections (26) which are parallel to each other and two semicircular arches (27) connecting the ends of the two straight sections (26), wherein, optionally, the distance of the straight sections (26) and/or a radius of the semicircular arches (27) decreases from bottom to top.

10. Well plate (11) of any of the preceding claims, **characterized in**
- **that** an angle (30) of inclination of the circumferential wall (16) with regard to the vertical direction (31) is from 15° to 65° or from 35° to 55°, and/or
- **that** the annular channel (15) is delimited at its outer circumference by walls (17, 18) which at least partially comprise an inclination angle (32) with regard to the vertical direction (31) towards the outside in a range from 15° to 65°or from 35° to 55°.

11. Well plate (11) of any of the preceding claims, **characterized in that** the annular channel (15) has a volume of 50 to 1,500 µl or of 100 to 250 µl.

12. Well plate (11) of any of the preceding claims, **characterized in that**, in a direction of the horizontal distance (28) of the at least two retaining elements (24), two electrode free spaces (25) are formed at the outer circumference of the at least one cavity (14).

13. Well plate (11) of any of the preceding claims, **characterized in that** a bottom of the annular channel (15) and the upper edge (22) of the circumferential wall (16) extend horizontally and that the at least one cavity (14) is closed except of its opening at the upper side of the plate main body (12).

14. Well plate (11) of any of the preceding claims, **characterized in that** the at least one cavity (14) is completely lined with biocompatible materials and/or that the circumferential wall (16) is made of a smooth and/or a hydrophobic material.

15. Well plate (11) of any of the preceding claims, **characterized in that**, in the well plate (11), a plurality of cavities (14) are arranged side-by-side in two horizontal directions.

16. Use of a well plate (11) of any of the preceding claims, **characterized in that** a suspension (38) of connective tissue cells and further cells is placed into the annular channel (15), that the cavity (14) is filled up with a culture medium (42) up to above the retaining elements (24), and that movements of a free upper end (44) of the at least one of the retaining elements (24) or of each retaining element (24) or of a pointer area (36) upwardly connecting thereto are monitored with a camera (45).

17. Use of claim 16, **characterized in**
- **that** the free upper end (44) of the pointer area (36) extends beyond the culture medium (42), and/or
- **that** the culture medium (42) is adapted to the or each retaining finger (21) in terms of color such that the or each retaining finger (21) is optically contrastive with regard to the culture medium (42).

18. Use of claim 16 or 17, **characterized in that** the further cells are selected from muscle cells, heart muscle cells or skeletal muscle cells.

19. Use of any of the claims 16 to 18, **characterized in that** the suspension further includes a hydrogel.

20. Use of any of the claims 16 to 19, **characterized in that** an electric voltage of varying amplitude is applied between electrodes at the retaining elements (24) and/or in the culture medium (42).

## Revendications

1. Plaque perforée (11) avec un corps principal de plaque (12) et avec au moins une cavité (14) dans un côté supérieur (13) du corps principal de plaque (12),
- dans laquelle, dans l'au moins une cavité (14), un canal annulaire ouvert en haut (15) est réalisé, qui est limité, au niveau de sa périphérie interne, par une paroi périphérique fermée (16),
**caractérisée en ce que**
- une périphérie externe horizontale de la paroi périphérique (16) diminue du bas vers le haut jusqu'à une arête supérieure (22) de la paroi périphérique (16) et
- à l'intérieur de la périphérie externe horizontale de la paroi périphérique (16), au niveau de son arête supérieure (22), se raccordent au moins deux éléments de maintien (24) vers le haut à l'arête supérieure (22) de la paroi périphérique (16),
- dans laquelle les au moins deux éléments de maintien (24) présentent une distance horizontale libre (28) entre eux et
- dans laquelle au moins un des deux éléments de maintien (24) s'appuie dans la direction horizontale de manière élastique contre le corps principal de plaque (12).

2. Plaque perforée (11) selon la revendication 1, **caractérisée en ce que** chacun des deux éléments de maintien (24) s'appuie, dans l'au moins une cavité (14), contre le corps principal de plaque (12).

3. Plaque perforée (11) selon l'une des revendications précédentes, **caractérisée en ce qu'**au moins un des éléments de maintien (24) ou chaque élément de maintien (24) fait respectivement partie d'un doigt de maintien (21) s'étendant le long de la verticale (31).

4. Plaque perforée (11) selon la revendication 3, **caractérisée en ce que** la paroi périphérique (16) entoure au moins une partie supérieure d'un trou borgne ouvert en haut (20), sur le fond (33) duquel le ou chaque doigt de maintien (21) est logé au niveau du corps principal de plaque (12), dans laquelle, en option, le ou chaque doigt de maintien (21) est formé d'une seule pièce avec une plaque de fond (34) qui est logée par force sur le fond (33) du trou borgne (20).

5. Plaque perforée (11) selon l'une des revendications 3 et 4, **caractérisée en ce que**
- le ou chaque doigt de maintien (21) est élastique en flexion et/ou
- les au moins deux éléments de maintien (24) s'appuient, au niveau de deux doigts de maintien (21) de manière élastique l'un contre l'autre de façon à ce qu'une force horizontale de 1 mN entre les au moins deux éléments de maintien (24) conduise à une déviation horizontale relative des extrémités libres (44) des deux doigts de maintien (21) de l'ordre de 20 à 1 000 µm ou de 50 à 500 µm et/ou de l'ordre de 1 à 50 % ou de 10 à 30 % d'une distance de repos des doigts de maintien et/ou
- le ou chaque doigt de maintien (21) présente un diamètre de 0,5 à 3,0 mm ou de 0,75 à 2,0 mm et/ou le ou chaque doigt de maintien (21) présente une longueur de 5 à 10 mm du fond (33) du trou borgne (20) jusqu'à l'élément de maintien (24) formé au niveau de celui-ci et/ou une longueur totale jusqu'à son extrémité libre (44) de 10 à 20 mm et/ou
- le matériau (35) du ou de chaque doigt de maintien (21) présente un module d'élasticité de l'ordre de 0,5 à 50 MPa ou de 1 à 10 MPa.

6. Plaque perforée (11) selon l'une des revendications 3 à 5, **caractérisée en ce que**
- le ou chaque doigt de maintien (21) s'étend vers le haut avec une partie d'aiguille (36) jusque au-delà de l'élément de maintien (24) et/ou
- le ou chaque doigt de maintien (21) se termine en dessous du côté supérieur (13) du corps principal de plaque (12) et/ou
- le ou chaque doigt de maintien (21) est constitué d'un matériau (35) contrastant optiquement par rapport au corps principal de plaque (12) et/ou fluorescent.

7. Plaque perforée (11) selon l'une des revendications 3 à 6, **caractérisée en ce que** les au moins deux éléments de maintien (24) sont réalisés au niveau de deux doigts de maintien (21), qui comprennent respectivement au moins une partie électro-conductrice s'étendant jusque dans l'élément de maintien, et **en ce que** les parties électro-conductrices des au moins deux doigts de maintien (21) peuvent être mises en contact électrique à partir du côté inférieur du corps principal de plaque (12).

8. Plaque perforée (11) selon l'une des revendications 3 à 7, **caractérisée en ce que** le ou chaque doigt de maintien (21) présente, au niveau de l'élément de maintien (24), une encoche ou un renflement horizontal (23, 51).

9. Plaque perforée (11) selon l'une des revendications précédentes, **caractérisée en ce que** la périphérie externe horizontale de la paroi périphérique (16) est constituée de deux parties droites (26) parallèles entre elles et de deux arcs de demi-cercle (27) reliant entre elles les extrémités des deux parties droites (26), dans laquelle, en option, la distance entre les parties droites (26) et/ou un rayon des arcs de demi-cercle (27) diminue du bas vers le haut.

10. Plaque perforée (11) selon l'une des revendications précédentes, **caractérisée en ce que**
- un angle d'inclinaison (30) de la paroi périphérique (16) par rapport à la verticale (31) est de 15° à 65° ou de 35° à 55° et/ou
- le canal annulaire (15) est limité, au niveau de sa périphérie externe, par des parois (17, 18), qui présentent au moins partiellement un angle d'inclinaison (32) par rapport à la verticale (31), vers l'extérieur, de l'ordre de 15° à 65° ou de 35° à 55°.

11. Plaque perforée (11) selon l'une des revendications précédentes, **caractérisée en ce que** le canal annulaire (15) présente un volume de 50 à 1 500 µl ou de 100 à 250 µl.

12. Plaque perforée (11) selon l'une des revendications précédentes, **caractérisée en ce que**, dans la direction de la distance horizontale (28) entre les au moins deux éléments de maintien (24), sont réalisés deux emplacements libres d'électrodes (25) sur la périphérie externe de l'au moins une cavité (14).

13. Plaque perforée (11) selon l'une des revendications précédentes, **caractérisée en ce qu'**un fond du canal annulaire (15) et l'arête supérieure (22) de la paroi périphérique (16) s'étendent horizontalement et **en ce que** l'au moins une cavité (14) est fermée jusqu'à son ouverture au niveau du côté supérieur du corps principal de plaque (12).

14. Plaque perforée (11) selon l'une des revendications précédentes, **caractérisée en ce que** l'au moins une cavité (14) est revêtue entièrement de matériaux biocompatibles et/ou **en ce que** la paroi périphérique (16) est constituée d'un matériau lisse et/ou hydrophobe.

15. Plaque perforée (11) selon l'une des revendications précédentes, **caractérisée en ce que**, dans la plaque perforée (11), sont juxtaposées plusieurs cavités (14) dans deux directions horizontales.

16. Utilisation d'une plaque perforée (11) selon l'une des revendications précédentes, **caractérisée en ce qu'**une suspension (38) de cellules de tissu conjonctif et d'autres cellules sont introduites dans le canal annulaire (15), **en ce que** la cavité (14) est remplie jusque au-dessus des éléments de maintien (24) d'une solution nutritive (42) et **en ce que** les mouvements d'une extrémité supérieure libre (44) d'au moins un des éléments de maintien (24) ou de chaque élément de maintien (24) ou d'une partie d'aiguille (36) s'y raccordant vers le haut sont détectés avec une caméra (45).

17. Utilisation selon la revendication 16, **caractérisée en ce que**
- l'extrémité supérieure libre (44) de la partie d'aiguille (36) dépasse de la solution nutritive (42) et/ou
- la solution nutritive (42) est adaptée, en ce qui concerne sa couleur, au ou à chaque doigt de maintien (21), de façon à ce que le ou chaque doigt de maintien (21) contraste optiquement par rapport à la solution nutritive (42).

18. Utilisation selon la revendication 16 ou 17, **caractérisée en ce que** les autres cellules sont sélectionnées parmi des cellules musculaires, des cellules de myocarde ou des cellules de muscles squelettiques.

19. Utilisation selon l'une des revendications 16 à 18, **caractérisée en ce que** la suspension comprend en outre un hydrogel.

20. Utilisation selon l'une des revendications 16 à 19, **caractérisée en ce qu'**une tension électrique d'amplitude variable est appliquée entre des électrodes au niveau des éléments de maintien (24) et/ou dans la solution nutritive (42).
